# EUROPEAN PATENT SPECIFICATION

(11) **EP 1 092 776 B1**
(45) Date of publication and mention of the grant of the patent: **07.07.2004**
(21) Application number: 00121763.7
(22) Date of filing: 05.10.2000
(51) Int. Cl.: C12N 15/52, C12N 9/00, C12P 13/06, C12P 13/08, C12P 13/14, C12R 1/19

(54) **Method for producing L-amino acids by fermentation of an Escherichia strain which is transformed with a pyruvate carboxylase-encoding gene from Bacilus subtilis**
Verfahren zur Herstellung von L-Aminosäuren mittels Fermentation eines Escherichia Stammes, transformiert mit einem Pyruvat-carboxylase Gen aus Bacillus subtilis
Procédé de préparation d'acides amines par fermentation d'une souche d'Escherichia contenant un gène de pyruvate carboxylase provenant de Bacillus subtilis

(30) Priority: 14.10.1999 RU 99121636
(43) Date of publication of application: 18.04.2001
(73) Proprietor: Ajinomoto Co., Inc., Tokyo (JP)
(72) Inventor: Gusyatiner, Mikhail Markovich, 113535 Moscow (RU); Kozlov, Yuri Ivanovich, 117574 Moscow (RU); Ptitsyn, Leonid Romanovich, 115404 Moscow (RU); Altman, Irina Borisovna, 119435 Moscow (RU); Voroshilova, Elvira Borisovna, 113535 Moscow (RU); Iomantas, Yurgis Antanas Vladovich, 127273 Moscow (RU); Yampolskaya, Tatyana Abramovna, 123364 Moscow (RU)
(74) Representative: Strehl Schübel-Hopf & Partner

(56) References cited:
- WO-A-99/18228
- WO-A-99/53035
- FR-A- 2 738 014
- US-A- 4 278 765
- PETERS-WENDISCH ET AL: "Pyruvate carboxylase as an anaplerotic enzyme in Corynebacterium glutamicum" MICROBIOLOGY, vol. 143, no. PART 04, April 1997 (1997-04), pages 1095-1103, XP002110209
- DATABASE EMBL [Online] EMBL; ID BSUB0008, AC Z99111, 20 November 1997 (1997-11-20) KUNST F ET AL.: "Bacillus subtilis complete genome (section 8 of 21); from 1394791 to 1603020" XP002160795
- KOFFAS ET AL: "Sequence of the Corynebacterium glutamicum pyruvate carboxylase gene" APPLIED MICROBIOLOGY AND BIOTECHNOLOGY, vol. 50, no. 3, September 1998 (1998-09), pages 346-352, XP002110411
- KRAMER R: "Genetic and physiological approaches for the production of amino acids" JOURNAL OF BIOTECHNOLOGY, vol. 45, no. 1, 12 February 1996 (1996-02-12), pages 1-21, XP004036833
- DELONG E.F. ET AL: 'Isolation of the lux genes from Photobacterium leiognathi and expression if Escherichia coli' GENE vol. 54, 1987, pages 203 - 210
- MANCINI J.A. ET AL: 'Cloning and expression of the Photobacterium phosphorum luminescence system demonstrates a unique lux gene organization' JOURNAL OF BIOLOGICAL CHEMISTRY vol. 263, no. 28, 05 October 1988, pages 14308 - 14314
- FRACKMAN S. ET AL: 'Cloning, organization, and expression of the bioluminescence genes of Xenorhabdus luminescens' JOURNAL OF BACTERIOLOGY vol. 172, no. 10, October 1990, pages 5767 - 5773

### SEQUENCE LISTING

<110> Ajinomoto Co., Inc.
<120> Method for Producing L- Amino Acid by Fermentation
<130> EPA-53466
<150> RU 99121636
<151> 1999-10-14
<160> 4
<170> PatentIn Ver. 2.0
<210> 1
<211> 20
<212> DNA
<213> Artificial Sequence
<220>
<223> Description of Artificial Sequence:primer
<400> 1
   gatataaggg gacttcagag 20
<210> 2
<211> 20
<212> DNA
<213> Artificial Sequence
<220>
<223> Description of Artificial Sequence:primer
<400> 2
<210> 3
<211> 3462
<212> DNA
<213> Bacillus subtilis
<220>
<221> CDS
<222> (16)..(3459)
<400> 3
   aagggggaga aaagt ttg tct cag caa tcg ata caa aaa gta tta gta gca  51
                    Leu Ser Gln Gln Ser Ile Gln Lys Val Leu Val Ala
                     1               5                  10
   aac agg gga gaa att gca atc cga ata ttc cgg gcg tgt acc gag ttg   99
   Asn Arg Gly Glu Ile Ala Ile Arg Ile Phe Arg Ala Cys Thr Glu Leu
           15                  20                  25
   aat att cgt aca gtt gcg gtc tat tca aaa gaa gat tcc ggt tcc tac    147
   Asn Ile Arg Thr Val Ala Val Tyr Ser Lys Glu Asp Ser Gly Ser Tyr
       30                  35                  40
   cat cgg tac aaa gcg gat gaa gca tac ttg gtc ggt gaa ggg aaa aaa   195
   His Arg Tyr Lys Ala Asp Glu Ala Tyr Leu Val Gly Glu Gly Lys Lys
   45                  50                  55                  60
   ccg att gat gct tac ctg gat att gaa ggt atc att gat att gcg aaa   243
   Pro Ile Asp Ala Tyr Leu Asp Ile Glu Gly Ile Ile Asp Ile Ala Lys
                   65                  70                  75
   aga aac aaa gtc gat gca att cat ccg gga tac ggt ttc tta tct gaa   291
   Arg Asn Lys Val Asp Ala Ile His Pro Gly Tyr Gly Phe Leu Ser Glu
               80                  85                  90
   aat att cat ttt gcg aga cga tgt gaa gaa gaa ggc atc gta ttc ata   339
   Asn Ile His Phe Ala Arg Arg Cys Glu Glu Glu Gly Ile Val Phe Ile
   95 100 105
   ggg cca aaa tcc gag cat ctc gat atg ttt ggt gac aag gta aaa gcg 387
   Gly Pro Lys Ser Glu His Leu Asp Met Phe Gly Asp Lys Val Lys Ala
   110 115 120
   cgt gag cag gca gaa aaa gcg gga atc ccc gtg att ccg gga agc gac 435
   Arg Glu Gln Ala Glu Lys Ala Gly Ile Pro Val Ile Pro Gly Ser Asp
   125 130 135 140
   ggt cct gcc gaa acg ctt gaa gcc gtc gaa caa ttt gga caa gct aac 483
   Gly Pro Ala Glu Thr Leu Glu Ala Val Glu Gln Phe Gly Gln Ala Asn
   145 150 155
   ggt tat ccg atc atc att aaa gcc tcg ctt ggc ggc ggc ggc cgc ggt 531
   Gly Tyr Pro Ile Ile Ile Lys Ala Ser Leu Gly Gly Gly Gly Arg Gly
   160 165 170
   atg cgg att gtc aga tct gaa agt gaa gtt aaa gaa gca tat gag cgt 579
   Met Arg Ile Val Arg Ser Glu Ser Glu Val Lys Glu Ala Tyr Glu Arg
   175 180 185
   gct aaa tca gag gcg aaa gca gcc ttt ggc aat gat gaa gtt tat gta 627
   Ala Lys Ser Glu Ala Lys Ala Ala Phe Gly Asn Asp Glu Val Tyr Val
   190 195 200
   gaa aaa tta att gag aat ccg aaa cat att gag gtt cag gtc att gga 675
   Glu Lys Leu Ile Glu Asn Pro Lys His Ile Glu Val Gln Val Ile Gly
   205 210 215 220
   gac aag cag ggc aat gtc gtc cat ctt ttt gag agg gat tgc tcc gtt 723
   Asp Lys Gln Gly Asn Val Val His Leu Phe Glu Arg Asp Cys Ser Val
   225 230 235
   caa aga cgc cat caa aaa gtc att gaa gtg gcg ccg agt gtc tcg ctg 771
   Gln Arg Arg His Gln Lys Val Ile Glu Val Ala Pro Ser Val Ser Leu
   240 245 250
   tca cct gaa tta agg gac caa att tgt gag gct gca gtt gcg ctt gcc 819
   Ser Pro Glu Leu Arg Asp Gln Ile Cys Glu Ala Ala Val Ala Leu Ala
   255 260 265
   aaa aat gta aac tat ata aat gcg ggg acg gtc gaa ttc ctt gtt gca 867
   Lys Asn Val Asn Tyr Ile Asn Ala Gly Thr Val Glu Phe Leu Val Ala
       270                 275                 280
   aac aac gag ttc tac ttt att gaa gta aat cct cgc gta caa gtt gaa    915
   Asn Asn Glu Phe Tyr Phe Ile Glu Val Asn Pro Arg Val Gln Val Glu
   285                 290                 295                 300
   cac acg ata aca gaa atg att act ggt gtc gat att gtt caa act cag    963
   His Thr Ile Thr Glu Met Ile Thr Gly Val Asp Ile Val Gln Thr Gln
                   305                 310                 315
   atc ctt gtt gcc caa ggg cac agc ctt cac agc aaa aaa gta aat att    1011
   Ile Leu Val Ala Gln Gly His Ser Leu His Ser Lys Lys Val Asn Ile
               320                 325                 330
   cct gag caa aag gac att ttt aca atc ggc tat gcc att cag tca cgg    1059
   Pro Glu Gln Lys Asp Ile Phe Thr Ile Gly Tyr Ala Ile Gln Ser Arg
           335                 340                 345
   gtt acg act gag gat ccg caa aat gat ttc atg cct gat aca gga aaa    1107
   Val Thr Thr Glu Asp Pro Gln Asn Asp Phe Met Pro Asp Thr Gly Lys
       350                 355                 360
   atc atg gct tac cgc tca ggc ggc ggt ttt ggt gtc cgt ctt gat acc    1155
   Ile Met Ala Tyr Arg Ser Gly Gly Gly Phe Gly Val Arg Leu Asp Thr
   365                 370                 375                 380
   gga aac agc ttc cag ggc gcc gtg atc aca cca tac tat gat tca ctt    1203
   Gly Asn Ser Phe Gln Gly Ala Val Ile Thr Pro Tyr Tyr Asp Ser Leu
                   385                 390                 395
   ctc gtt aag ctt tca act tgg gct tta acg ttt gaa cag gca gct gcc    1251
   Leu Val Lys Leu Ser Thr Trp Ala Leu Thr Phe Glu Gln Ala Ala Ala
               400                 405                 410
   aaa atg gtg cga aac ctt cag gag ttt aga atc aga ggc ata aaa acg    1299
   Lys Met Val Arg Asn Leu Gln Glu Phe Arg Ile Arg Gly Ile Lys Thr
           415                 420                 425
   aac att ccg ttc ctt gag aac gtt gca aag cat gag aag ttc ctg aca    1347
   Asn Ile Pro Phe Leu Glu Asn Val Ala Lys His Glu Lys Phe Leu Thr
       430                 435                 440
   ggg caa tat gat aca tct ttc att gat aca acg cct gaa tta ttt aat    1395
   Gly Gln Tyr Asp Thr Ser Phe Ile Asp Thr Thr Pro Glu Leu Phe Asn
   445                 450                 455                 460
   ttc cct aaa caa aaa gac cgc gga acg aaa atg ctc act tac atc ggc 1443
   Phe Pro Lys Gln Lys Asp Arg Gly Thr Lys Met Leu Thr Tyr Ile Gly
   465 470 475
   aat gtg aca gtg aac ggc ttc cct gga atc ggg aaa aaa gaa aaa ccg 1491
   Asn Val Thr Val Asn Gly Phe Pro Gly Ile Gly Lys Lys Glu Lys Pro
   480 485 490
   gcg ttt gac aaa ccg tta ggc gta aag gta gac gtt gat cag cag cct 1539
   Ala Phe Asp Lys Pro Leu Gly Val Lys Val Asp Val Asp Gln Gln Pro
   495 500 505
   gcc aga gga aca aag caa att ctc gat gaa aaa ggt gca gaa ggg ctt 1587
   Ala Arg Gly Thr Lys Gln Ile Leu Asp Glu Lys Gly Ala Glu Gly Leu
   510 515 520
   gca aat tgg gtt aag gag cag aaa tct gtc ctt tta act gat acg aca 1635
   Ala Asn Trp Val Lys Glu Gln Lys Ser Val Leu Leu Thr Asp Thr Thr
   525 530 535 540
   ttc agg gat gcc cac caa tcg tta ttg gca act aga atc aga tcg cat 1683
   Phe Arg Asp Ala His Gln Ser Leu Leu Ala Thr Arg Ile Arg Ser His
   545 550 555
   gat ttg aaa aaa atc gca aat ccg acg gct gcg tta tgg cct gaa cta 1731
   Asp Leu Lys Lys Ile Ala Asn Pro Thr Ala Ala Leu Trp Pro Glu Leu
   560 565 570
   ttc agt atg gaa atg tgg gga ggc gcg acc ttc gat gta gcc tac cga 1779
   Phe Ser Met Glu Met Trp Gly Gly Ala Thr Phe Asp Val Ala Tyr Arg
   575 580 585
   ttc ctg aaa gaa gat ccg tgg aaa cgt ttg gaa gat ctt cgc aaa gaa 1827
   Phe Leu Lys Glu Asp Pro Trp Lys Arg Leu Glu Asp Leu Arg Lys Glu
   590 595 600
   gtg ccg aat acc tta ttc cag atg ttg ctt cgc tca tca aat gcg gtc 1875
   Val Pro Asn Thr Leu Phe Gln Met Leu Leu Arg Ser Ser Asn Ala Val
   605 610 615 620
   ggc tat acg aat tat ccg gac aat gtg att aaa gaa ttt gtg aag caa 1923
   Gly Tyr Thr Asn Tyr Pro Asp Asn Val Ile Lys Glu Phe Val Lys Gln
   625 630 635
   tca gct caa tcc ggt att gat gtg ttt cgt att ttc gac agc tta aac 1971
   Ser Ala Gln Ser Gly Ile Asp Val Phe Arg Ile Phe Asp Ser Leu Asn
   640 645 650
   tgg gta aaa ggg atg acg tta gcc att gat gct gtt agg gat acc ggc 2019
   Trp Val Lys Gly Met Thr Leu Ala Ile Asp Ala Val Arg Asp Thr Gly
   655 660 665
   aaa gtg gca gaa gct gcg att tgt tat acg gga gat atc ctt gac aag 2067
   Lys Val Ala Glu Ala Ala Ile Cys Tyr Thr Gly Asp Ile Leu Asp Lys
   670 675 680
   aac cgg acg aag tac gac ctt gca tat tat aca tcg atg gcg aag gag 2115
   Asn Arg Thr Lys Tyr Asp Leu Ala Tyr Tyr Thr Ser Met Ala Lys Glu
   685 690 695 700
   ctt gag gcg gcc gga gcc cat att ctc ggg att aaa gat atg gca ggg 2163
   Leu Glu Ala Ala Gly Ala His Ile Leu Gly Ile Lys Asp Met Ala Gly
   705 710 715
   ctg tta aaa ccg cag gct gca tat gag ctc gtt tct gcg ttg aaa gaa 2211
   Leu Leu Lys Pro Gln Ala Ala Tyr Glu Leu Val Ser Ala Leu Lys Glu
   720 725 730
   acg atc gac att ccg gtt cac ctt cat acg cat gat acg agc gga aac 2259
   Thr Ile Asp Ile Pro Val His Leu His Thr His Asp Thr Ser Gly Asn
   735 740 745
   ggt att tat atg tat gcg aaa gct gtt gaa gcc ggc gtt gat atc ata 2307
   Gly Ile Tyr Met Tyr Ala Lys Ala Val Glu Ala Gly Val Asp Ile Ile
   750 755 760
   gac gtg gcg gtc agc tca atg gcg gga tta acg tca cag cct agc gcg 2355
   Asp Val Ala Val Ser Ser Met Ala Gly Leu Thr Ser Gln Pro Ser Ala
   765 770 775 780
   agc gga ttt tat cat gcg atg gaa ggc aac gac cgc cgt ccg gaa atg 2403
   Ser Gly Phe Tyr His Ala Met Glu Gly Asn Asp Arg Arg Pro Glu Met
   785 790 795
   aat gtc caa ggc gtt gaa ttg ctg tcc caa tat tgg gag tcg gtg cgt 2451
   Asn Val Gln Gly Val Glu Leu Leu Ser Gln Tyr Trp Glu Ser Val Arg
   800 805 810
   aaa tat tat agt gaa ttt gaa agc gga atg aag tct ccg cat act gaa 2499
   Lys Tyr Tyr Ser Glu Phe Glu Ser Gly Met Lys Ser Pro His Thr Glu
   815 820 825
   att tat gaa cac gaa atg cca ggg ggc caa tac agc aac ctg cag cag 2547
   Ile Tyr Glu His Glu Met Pro Gly Gly Gln Tyr Ser Asn Leu Gln Gln
   830 835 840
   caa gcc aag gga gta ggc ctt ggc gac cgc tgg aac gaa gtc aag gaa 2595
   Gln Ala Lys Gly Val Gly Leu Gly Asp Arg Trp Asn Glu Val Lys Glu
   845 850 855 860
   atg tac aga cgc gtg aac gat atg ttc ggt gac atc gtc aag gta acg 2643
   Met Tyr Arg Arg Val Asn Asp Met Phe Gly Asp Ile Val Lys Val Thr
   865 870 875
   cct tcc tca aaa gta gtc gga gat atg gca ctc tac atg gtg caa aac 2691
   Pro Ser Ser Lys Val Val Gly Asp Met Ala Leu Tyr Met Val Gln Asn
   880 885 890
   aat ctg act gaa aaa gac gtt tac gaa aaa ggt gaa tct tta gat ttc 2739
   Asn Leu Thr Glu Lys Asp Val Tyr Glu Lys Gly Glu Ser Leu Asp Phe
   895 900 905
   cct gat tct gtc gtg gag ctt ttt aaa gga aat atc ggc cag cct cat 2787
   Pro Asp Ser Val Val Glu Leu Phe Lys Gly Asn Ile Gly Gln Pro His
   910 915 920
   ggc gga ttc cca gaa aaa ctg caa aag ctg atc tta aaa ggg cag gag 2835
   Gly Gly Phe Pro Glu Lys Leu Gln Lys Leu Ile Leu Lys Gly Gln Glu
   925 930 935 940
   ccg att aca gtc aga ccg ggc gaa ctg ctt gag ccg gtg tca ttt gaa 2883
   Pro Ile Thr Val Arg Pro Gly Glu Leu Leu Glu Pro Val Ser Phe Glu
   945 950 955
   gcg atc aaa cag gaa ttt aaa gag cag cat aac ttg gaa att tca gat 2931
   Ala Ile Lys Gln Glu Phe Lys Glu Gln His Asn Leu Glu Ile Ser Asp
   960 965 970
   cag gat gct gtg gca tat gcc ctt tat cct aaa gtc ttc act gat tat 2979
   Gln Asp Ala Val Ala Tyr Ala Leu Tyr Pro Lys Val Phe Thr Asp Tyr
   975 980 985
   gtg aaa acg aca gaa agc tat gga gac atc tcg gta tta gat aca ccg 3027
   Val Lys Thr Thr Glu Ser Tyr Gly Asp Ile Ser Val Leu Asp Thr Pro
   990 995 1000
   aca ttc ttc tac ggt atg aca tta ggt gaa gag ata gaa gtt gaa att 3075
   Thr Phe Phe Tyr Gly Met Thr Leu Gly Glu Glu Ile Glu Val Glu Ile
   1005 1010 1015 1020
   gag cgc ggc aaa acg ctg atc gtt aag ctg att tca atc ggt gag cct 3123
   Glu Arg Gly Lys Thr Leu Ile Val Lys Leu Ile Ser Ile Gly Glu Pro
   1025 1030 1035
   cag cct gat gcc acc cgc gtc gtt tat ttc gaa ctc aac ggg cag ccg 3171
   Gln Pro Asp Ala Thr Arg Val Val Tyr Phe Glu Leu Asn Gly Gln Pro
   1040 1045 1050
   cgt gaa gta gtc att aaa gat gaa agc att aag tct tcc gtt cag gaa 3219
   Arg Glu Val Val Ile Lys Asp Glu Ser Ile Lys Ser Ser Val Gln Glu
   1055 1060 1065
   agg ctg aaa gca gac cgg aca aat cca agc cac atc gca gct tcc atg 3267
   Arg Leu Lys Ala Asp Arg Thr Asn Pro Ser His Ile Ala Ala Ser Met
   1070 1075 1080
   cct gga aca gtt att aag gta ttg gct gaa gca ggc aca aaa gtc aat 3315
   Pro Gly Thr Val Ile Lys Val Leu Ala Glu Ala Gly Thr Lys Val Asn
   1085 1090 1095 1100
   aaa ggt gat cat ttg atg att aat gaa gcg atg aaa atg gaa aca acg 3363
   Lys Gly Asp His Leu Met Ile Asn Glu Ala Met Lys Met Glu Thr Thr
   1105 1110 1115
   gtt cag gcg cct ttc tca gga aca atc aag cag gtt cat gtg aaa aat 3411
   Val Gln Ala Pro Phe Ser Gly Thr Ile Lys Gln Val His Val Lys Asn
   1120 1125 1130
   ggt gag ccg atc caa acg gga gat ctg ctc ctt gaa att gaa aaa gca 3459
   Gly Glu Pro Ile Gln Thr Gly Asp Leu Leu Leu Glu Ile Glu Lys Ala
   1135 1140 1145
   taa 3462
<210> 4
<211> 1148
<212> PRT
<213> Bacillus subtilis
<400> 4
   Leu Ser Gln Gln Ser Ile Gln Lys Val Leu Val Ala Asn Arg Gly Glu
   1 5 10 15
   Ile Ala Ile Arg Ile Phe Arg Ala Cys Thr Glu Leu Asn Ile Arg Thr
   20 25 30
   Val Ala Val Tyr Ser Lys Glu Asp Ser Gly Ser Tyr His Arg Tyr Lys
   35 40 45
   Ala Asp Glu Ala Tyr Leu Val Gly Glu Gly Lys Lys Pro Ile Asp Ala
   50 55 60
   Tyr Leu Asp Ile Glu Gly Ile Ile Asp Ile Ala Lys Arg Asn Lys Val
   65 70 75 80
   Asp Ala Ile His Pro Gly Tyr Gly Phe Leu Ser Glu Asn Ile His Phe
   85 90 95
   Ala Arg Arg Cys Glu Glu Glu Gly Ile Val Phe Ile Gly Pro Lys Ser
   100 105 110
   Glu His Leu Asp Met Phe Gly Asp Lys Val Lys Ala Arg Glu Gln Ala
   115 120 125
   Glu Lys Ala Gly Ile Pro Val Ile Pro Gly Ser Asp Gly Pro Ala Glu
   130 135 140
   Thr Leu Glu Ala Val Glu Gln Phe Gly Gln Ala Asn Gly Tyr Pro Ile
   145 150 155 160
   Ile Ile Lys Ala Ser Leu Gly Gly Gly Gly Arg Gly Met Arg Ile Val
   165 170 175
   Arg Ser Glu Ser Glu Val Lys Glu Ala Tyr Glu Arg Ala Lys Ser Glu
   180 185 190
   Ala Lys Ala Ala Phe Gly Asn Asp Glu Val Tyr Val Glu Lys Leu Ile
   195 200 205
   Glu Asn Pro Lys His Ile Glu Val Gln Val Ile Gly Asp Lys Gln Gly
   210 215 220
   Asn Val Val His Leu Phe Glu Arg Asp Cys Ser Val Gln Arg Arg His
   225 230 235 240
   Gln Lys Val Ile Glu Val Ala Pro Ser Val Ser Leu Ser Pro Glu Leu
   245 250 255
   Arg Asp Gln Ile Cys Glu Ala Ala Val Ala Leu Ala Lys Asn Val Asn
   260 265 270
   Tyr Ile Asn Ala Gly Thr Val Glu Phe Leu Val Ala Asn Asn Glu Phe
   275 280 285
   Tyr Phe Ile Glu Val Asn Pro Arg Val Gln Val Glu His Thr Ile Thr
   290 295 300
   Glu Met Ile Thr Gly Val Asp Ile Val Gln Thr Gln Ile Leu Val Ala
   305 310 315 320
   Gln Gly His Ser Leu His Ser Lys Lys Val Asn Ile Pro Glu Gln Lys
   325 330 335
   Asp Ile Phe Thr Ile Gly Tyr Ala Ile Gln Ser Arg Val Thr Thr Glu
   340 345 350
   Asp Pro Gln Asn Asp Phe Met Pro Asp Thr Gly Lys Ile Met Ala Tyr
   355 360 365
   Arg Ser Gly Gly Gly Phe Gly Val Arg Leu Asp Thr Gly Asn Ser Phe
   370 375 380
   Gln Gly Ala Val Ile Thr Pro Tyr Tyr Asp Ser Leu Leu Val Lys Leu
   385 390 395 400
   Ser Thr Trp Ala Leu Thr Phe Glu Gln Ala Ala Ala Lys Met Val Arg
   405 410 415
   Asn Leu Gln Glu Phe Arg Ile Arg Gly Ile Lys Thr Asn Ile Pro Phe
   420 425 430
   Leu Glu Asn Val Ala Lys His Glu Lys Phe Leu Thr Gly Gln Tyr Asp
   435 440 445
   Thr Ser Phe Ile Asp Thr Thr Pro Glu Leu Phe Asn Phe Pro Lys Gln
   450 455 460
   Lys Asp Arg Gly Thr Lys Met Leu Thr Tyr Ile Gly Asn Val Thr Val
   465 470 475 480
   Asn Gly Phe Pro Gly Ile Gly Lys Lys Glu Lys Pro Ala Phe Asp Lys
   485 490 495
   Pro Leu Gly Val Lys Val Asp Val Asp Gln Gln Pro Ala Arg Gly Thr
   500 505 510
   Lys Gln Ile Leu Asp Glu Lys Gly Ala Glu Gly Leu Ala Asn Trp Val
   515 520 525
   Lys Glu Gln Lys Ser Val Leu Leu Thr Asp Thr Thr Phe Arg Asp Ala
   530 535 540
   His Gln Ser Leu Leu Ala Thr Arg Ile Arg Ser His Asp Leu Lys Lys
   545 550 555 560
   Ile Ala Asn Pro Thr Ala Ala Leu Trp Pro Glu Leu Phe Ser Met Glu
   565 570 575
   Met Trp Gly Gly Ala Thr Phe Asp Val Ala Tyr Arg Phe Leu Lys Glu
   580 585 590
   Asp Pro Trp Lys Arg Leu Glu Asp Leu Arg Lys Glu Val Pro Asn Thr
   595 600 605
   Leu Phe Gln Met Leu Leu Arg Ser Ser Asn Ala Val Gly Tyr Thr Asn
   610 615 620
   Tyr Pro Asp Asn Val Ile Lys Glu Phe Val Lys Gln Ser Ala Gln Ser
   625 630 635 640
   Gly Ile Asp Val Phe Arg Ile Phe Asp Ser Leu Asn Trp Val Lys Gly
   645 650 655
   Met Thr Leu Ala Ile Asp Ala Val Arg Asp Thr Gly Lys Val Ala Glu
   660 665 670
   Ala Ala Ile Cys Tyr Thr Gly Asp Ile Leu Asp Lys Asn Arg Thr Lys
   675 680 685
   Tyr Asp Leu Ala Tyr Tyr Thr Ser Met Ala Lys Glu Leu Glu Ala Ala
   690 695 700
   Gly Ala His Ile Leu Gly Ile Lys Asp Met Ala Gly Leu Leu Lys Pro
   705 710 715 720
   Gln Ala Ala Tyr Glu Leu Val Ser Ala Leu Lys Glu Thr Ile Asp Ile
   725 730 735
   Pro Val His Leu His Thr His Asp Thr Ser Gly Asn Gly Ile Tyr Met
   740 745 750
   Tyr Ala Lys Ala Val Glu Ala Gly Val Asp Ile Ile Asp Val Ala Val
   755 760 765
   Ser Ser Met Ala Gly Leu Thr Ser Gln Pro Ser Ala Ser Gly Phe Tyr
   770 775 780
   His Ala Met Glu Gly Asn Asp Arg Arg Pro Glu Met Asn Val Gln Gly
   785 790 795 800
   Val Glu Leu Leu Ser Gln Tyr Trp Glu Ser Val Arg Lys Tyr Tyr Ser
   805 810 815
   Glu Phe Glu Ser Gly Met Lys Ser Pro His Thr Glu Ile Tyr Glu His
   820 825 830
   Glu Met Pro Gly Gly Gln Tyr Ser Asn Leu Gln Gln Gln Ala Lys Gly
   835 840 845
   Val Gly Leu Gly Asp Arg Trp Asn Glu Val Lys Glu Met Tyr Arg Arg
   850 855 860
   Val Asn Asp Met Phe Gly Asp Ile Val Lys Val Thr Pro Ser Ser Lys
   865 870 875 880
   Val Val Gly Asp Met Ala Leu Tyr Met Val Gln Asn Asn Leu Thr Glu
   885 890 895
   Lys Asp Val Tyr Glu Lys Gly Glu Ser Leu Asp Phe Pro Asp Ser Val
   900 905 910
   Val Glu Leu Phe Lys Gly Asn Ile Gly Gln Pro His Gly Gly Phe Pro
   915 920 925
   Glu Lys Leu Gln Lys Leu Ile Leu Lys Gly Gln Glu Pro Ile Thr Val
   930 935 940
   Arg Pro Gly Glu Leu Leu Glu Pro Val Ser Phe Glu Ala Ile Lys Gln
   945 950 955 960
   Glu Phe Lys Glu Gln His Asn Leu Glu Ile Ser Asp Gln Asp Ala Val
   965 970 975
   Ala Tyr Ala Leu Tyr Pro Lys Val Phe Thr Asp Tyr Val Lys Thr Thr
   980 985 990
   Glu Ser Tyr Gly Asp Ile Ser Val Leu Asp Thr Pro Thr Phe Phe Tyr
   995 1000 1005
   Gly Met Thr Leu Gly Glu Glu Ile Glu Val Glu Ile Glu Arg Gly Lys
   1010 1015 1020
   Thr Leu Ile Val Lys Leu Ile Ser Ile Gly Glu Pro Gln Pro Asp Ala
   025 1030 1035 1040
   Thr Arg Val Val Tyr Phe Glu Leu Asn Gly Gln Pro Arg Glu Val Val
   1045 1050 1055
   Ile Lys Asp Glu Ser Ile Lys Ser Ser Val Gln Glu Arg Leu Lys Ala
   1060 1065 1070
   Asp Arg Thr Asn Pro Ser His Ile Ala Ala Ser Met Pro Gly Thr Val
   1075 1080 1085
   Ile Lys Val Leu Ala Glu Ala Gly Thr Lys Val Asn Lys Gly Asp His
   1090 1095 1100
   Leu Met Ile Asn Glu Ala Met Lys Met Glu Thr Thr Val Gln Ala Pro
   105 1110 1115 1120
   Phe Ser Gly Thr Ile Lys Gln Val His Val Lys Asn Gly Glu Pro Ile
   1125 1130 1135
   Gln Thr Gly Asp Leu Leu Leu Glu Ile Glu Lys Ala
   1140 1145

## Description

### Technical Field

The present invention relates to a method for producing L-amino acid, specifically relates to a method for producing L-threonine, L-glutamic acid, L-homoserine, L-methionine, L-arginine, L-proline or L-isoleucine using a bacterium belonging to the genus Escherichia.

### Background Art

L-amino acids such as L-threonine and L-glutamic acid have conventionally been produced by fermentation methods principally utilizing coryneform bacteria which belong to the genera *Brevibacterium, Corynebacterium,* and *Microbacterium* or mutants thereof ("Amino Acid Fermentation", Gakkai Shuppan Center, pp.195-215, 1986).

On the other hand, there has been disclosed techniques for breeding of L-amino acid-producing bacteria belonging to the genus *Escherichia* utilizing genetic recombination techniques.

For example, there has been disclosed a method of producing L-lysine using *Escherichia coli* in which genes coding for dihydrodipicolinate synthetase, aspartokinase are desensitized to feedback inhibition by L-lysine and L-threonine, and diaminopimelate dehydrogenase (or tetrahydrodipicolinate succinylase and succinyldiaminopimelate deacylase) are enhanced (WO 95/16042).

Though the productivity of L-amino acids has considerably been improved by breeding of such microorganisms as mentioned above or production processes have been improved, it is still desired to develop more efficient processes for producing L-amino acids in order to meet the expected markedly increased future demand of the amino acids.

Pyruvate carboxylase [pyruvate:carbon dioxide ligase (ADP-forming), EC 6.4.1.1] is a biotine containing enzyme. It catalyses the carboxylation of pyruvate to form oxaloacetate. Pyruvate carboxylase has not been known in *Escherichia coli*, but *Bacillus* subtilis (Diesterhaft, M.D. and Freese, E., J. Biol. Chem., 248, No.17, 6062-6070 (1973)) and *Corynebacterium glutamicum* (Peters-Wendisch, P. G. et al., *Microbiology*, *144*, 915-927 (1998)) have pyruvate carboxylase and the nucleotide sequences of the genes coding for the enzymes have been reported.

Further, there has been known *Corynebacterium glutamicum* in which an activity of pyruvate carboxylase is enhanced or *Corynebacterium glutamicum* of which pyruvate carboxylase gene is inactivated (Peters-Wendisch, P. G. et al., Microbiology, 144, 915-927 (1998)).

WO 99/18228 discloses enhanced production of L-threonine, L-homoserine and L-glutamic acid by over-expression of the pyruvate carboxylase-encoding gene of *Corynebacterium glutamicum*. WO 99/53035 discloses that the expression of the pyruvate carboxylase-encoding gene from *Rhizobium etli* causes increased threonine production in *Escherichia coli*.

### Summary of the Invention

The present invention has been accomplished from the aforementioned point of view, and its object is to provide a method for producing L-amino acid, especially L-threonine, L-glutamic acid, or L-homoserine in high efficiency.

As a result of diligent investigation in order to achieve the object described above, the present inventors have found that introduction of a gene coding for pyruvate carboxylase (hereinafter referred to as *"pyc gene"*) into a bacterium belonging to the genus *Escherichia* can improve L-amino acid productivity of the bacterium. Thus, the present invention has been completed.

That is, the present invention is a bacterium belonging to the genus *Escherichia* which is transformed with a gene coding for pyruvate carboxylase and has L-amino acid productivity wherein the pyruvate carboxylase comprises the amino acid sequence of SEQ ID NO: 4.

The present invention also provides the bacterium described above wherein the gene coding for pyruvate carboxylase is derived from a bacterium belonging to the genus *Bacillus*.

The present invention further provides the bacterium described above wherein the gene coding for pyruvate carboxylase is introduced into said bacterium in a low copy number.

The present invention still further provides the bacterium described above wherein the L-amino acid is selected from the group consisting of L-threonine, L-glutamic acid, and L-homoserine.

The present invention also provides a method for producing L-amino acid comprising cultivating the above bacterium in a medium, producing and accumulating the L-amino acid in the medium, and collecting the L-amino acid from the medium.

The present invention further provides the method described above wherein the L-amino acid is selected the group consisting of L-threonine, L-glutamic acid, and L-homoserine.

The term "L-amino acid productivity" means a property that a bacterium produces and accumulates the L-amino acid in a medium in a larger amount than a wild type strain thereof.

According to the present invention, productivity of L-amino acid such as L-threonine, L-glutamic acid, or L-homoserine can be improved in a bacterium belonging to the genus *Escherichia*. Further, the present invention may be utilized to breeding of an L-amino acid producer belonging to the genus *Escherichia*.

### Brief Description of the Drawings

Figure 1 shows a scheme of constructing *Bacillus sutilis* pycA::KmR recipient strain for cloning *pycA* gene,
Figure 2 shows a scheme of cloning *pycA* gene from *Bacillus subtilis* 168, and
Figure 3 shows a scheme of construction of the plasmid containing *pycA* gene.

### Detailed Description of the Invention

The present invention will be explained in detail below.

### <1> A bacterium belonging to the genus Escherichia transformed with pyc gene

A bacterium belonging to the genus *Escherichia* of the present invention is a bacterium transformed with pyc gene. As the bacterium belonging to the genus *Escherichia*, there exemplified by *Escherichia coli*. While the *pyc* gene used in the present invention is not particularly limited so long as it can code a protein having pyruvate carboxylase activity, examples of the pyc gene include, for example, *pyc* gene derived from bacteria belonging to the genus *Bacillus* or pyc gene from coryneform bacteria. The nucleotide sequences of the *pyc* gene derived from *Bacillus subtilis* (Genbank/EMBL/DDBJ Accession Z97025, NID g2224758) and *Corynebacterium glutamicum* (Peters-Wendisch, P. G. et al., Microbiology, 144, 915-927 (1998)) has been reported. Therefore, *pyc* gene can be obtained by PCR (polymerase chain reaction: White,T.J. et al., Trends Genet., 5,185 (1989)) using primers synthesized according to the nucleotide sequences from chromosomal DNA of bacteria belonging to the genus *Bacillus* such as *Bacillus subtilis* or coryneform bacteria such as *Corynebacterium glutamicum* as a template.

A transformant of Escherichia coli 44/pMW119-pycA, which harbors pMW119-pycA containing pyc gene of *Bacillus subtilis* has been deposited in Russian National Collection of Industrial Microorganisms (VKPM) Depositary, GNIIgenetika; 1, Dorozhny Proezd., 1, 113545, Moscow, Russia, since August 30, 1999 under a Registration number of VKPM B-7822, and transferred from the original deposit to international deposit based on Budapest Treaty on September 1, 2000.

A transformant of Escherichia coli MG442/pMW119-pycA, which harbors pMW119-pycA containing pyc gene of *Bacillus subtilis* has been deposited in Russian National Collection of Industrial Microorganisms (VKPM) Depositary, GNIIgenetika; 1, Dorozhny Proezd., 1, 113545, Moscow, Russia, since August 30, 1999 under a Registration number of VKPM B-7821, and transferred from the original deposit to international deposit based on Budapest Treaty on September 1, 2000.

Incidentally, the gene coding pyruvate carboxylase of *Bacillus subtilis* (*pycA* gene) has been cloned as described below in a process of accomplishing the present invention.

First, a *pycA* gene-deficient strain of *Bacillus subtilis* was constructed. Then, using the strain as a recipient, *pycA* gene was cloned by isolating a DNA fragment which complemented citrate or aspartate auxotrophy of the strain from a genomic DNA library of a wild type strain of *Bacillus subtilis*. Since *Bacillus subtilis* forms oxaloacetate by pyruvate carboxylase, *Bacillus subtilis* cannot grow on minimal medium if it is deficient in the enzyme. However the growth is repaired by addition of L-aspartate, L-glutamate, citrate or succinate. The nucleotide sequence of *pycA* gene and the amino acid sequence coded by the gene are shown in SEQ ID Nos: 3 and 4.

The *pycA* gene-deficient strain is obtained, for example, as described below. A partial DNA frament of *ylaP* gene located immediately after the *pycA* gene on the chromosome is obtained by PCR. As primers for PCR are exemplified by oligonucleotides of SEQ ID NOs:1 and 2. Then a wild type strain of *Bacillus subtilis* is transformed with a plasmid DNA containing the obtained DNA fragment and a marker gene, followed by integrating the plasmid DNA into *ylaP* gene on the chromosomal DNA by homologous recombination. Next, chromosomal DNA library of the obtained strain is constructed using a restriction enzyme which recognizes a restriction site inside of *pycA* gene, for example, *PstI*. The recombinant plasmid containing the marker gene and a partial pycA gene fragment may be obtained by tranforming a wild type strain of *Bacillus subtilis* with the library and selecting clones expressing the marker gene. Then a wild type strain of *Bacillus subtilis* is transformed with the linearized recombinant plasmid and clones expressing the marker gene are selected to obtain *pycA* gene-deficient strain in which the plasmid DNA is integrated in *pycA* gene on chromosomal DNA of the strain.

Transformation of a bacterium belonging to the genus *Escherichia* with *pyc* gene may be performed by inserting *pyc* gene into an appropriate vector which functions in a bacterium belonging to the genus *Escherichia* to construct a recombinant vector, and introducing the recombinant vector to a bacterium belonging to the genus *Escherichia*.

The vector is exemplified by plasmid such as pBR322, pMW118, pMW119, pUC18, pUC19 or the like, phage vector such as λ1059, λBF101, M13mp9 or the like, and transposon such as Mu, Tn10, Tn5 or the like. However, in the present invention, a low copy plasmid such as pMW118 and pMW119 is preferable, because a transformant harboring a vector may be unstable if a high copy plasmid is used to introduce *pycA* gene as a vector.

The introduction of a DNA into a bacterium belonging to the genus *Escherichia* can be performed, for example, by a method of D. A. Morrison (Methods in Enzymology 68, 326 (1979)) or a method in which recipient bacterial cells are treated with calcium chloride to increase permeability of DNA (Mandel, M. and Higa, A., J. Mol. Biol., 53, 159 (1970)) and the like.

Preparation of the genomic DNA, construction of genomic DNA library, hybridization, PCR, preparation of plasmid DNA, digestion and ligation of DNA, transformation and the like are performed according to the methods which are known to a person skilled in the art. Such methods were described by Sambrook, J., Fritsche, E. F., Maniatis, T. in Molecular Cloning, Cold Spring Harbor Laboratory Press, 1.21 (1989).

L-amino acid productivity of a bacterium belonging to the genus *Escherichia* can be improved by conferring or enhancing pyruvate carboxylase activity on the bacterium through transforming the bacterium with *pyc* gene. This is probably because oxaloacetic acid is formed not only from phosphoenolpyruvic acid but also from pyruvic acid. That is, transport of glucose (or sucrose) molecule to a bacterial cell which is mediated by phosphoenolpyruvic acid system proceeds by consuming one molecule of phosphoenolpyruvic acid being accompnied with formation of one molecule of pyruvic acid. Although formed pyruvic acid is not directly utilized for L-amino acid synthesis in a bacterial cell, L-amino acid productivity may be increased by formation fo oxaloacetic acid from pyruvic acid.

As a bacterium belonging to the genus *Escherichia* to which *pyc* gene is to be introduced, a strain having an ability to produce objective L-amino acid is used. Alternatively, a bacterium belonging to the genus *Escherichia* which is transformed with *pyc* gene may be conferred L-amino acid productivity. Incidentally, *pyc* gene of *Escherichia coli* has not been known, however, if the gene is found in *E*. *coli* hereafter, the gene may be used.

Examples of L-amino acid-producing bacteria belonging to the genus *Escherichia* are described below.

### (1) L-threonine-producing bacteria

The L-threonine-producing bacteria belonging to the genus *Escherichia* may be exemplified by strain MG442 (deposited in Russian National Collection of Industrial Microorganisms (VKPM) Depositary, GNIIgenetika; 1, Dorozhny Proezd., 1, 113545, Moscow, Russia, under a Registration number of VKPM B-1628)(USP 4,278,765; Guayatiner M.M. et al., *Genetika* (in Russian), *14*, 947-956 (1978)). The strain MG442 had been obtained as a mutant resistant to L-threonine analogue, 2-amino-3-hydroxyvaleric acid, it was also an isoleucine auxotroph of leaky type. It produces L-threonine and L-glutamate as a by-product.

Alternatively, a transformant of MG442 which is transformed with pVIC40 (USP 5,175,107) may be preferably used as L-threonine-producing strain.

### (2) L-Lysine-producing bacteria

The L-lysine-producing bacteria belonging to the genus *Escherichia* may be exemplified by various bacterium described in WO95/16042. The L-lysine-producing bacteria are concretely exemplified by a bacterium in which aspartokinase activity and dihydrodipicolinate reductase activity are increased such as W3110(tyrA)RSFD80+pdapB.

### (3) L-homoserine-producing bacteria

The L-homoserine producing bacteria belonging to the genus *Escherichia* may be exemplified by strain 44 (*thrB*). This strain was derived from the known strain C600 (*thrB*, *leuB*) (Appleyard R.K., Genetics, 39, 440-452 (1954)) as Leu⁺ revertant. A transformant strain of 44 being transformed with a plasmid containing a *thrA* gene coding for aspartokinase-homoserine dehydrogenase I may be preferably used.

A strain of Escherichia coli 44 has been deposited in Russian National Collection of Industrial Microorganisms (VKPM) Depositary, GNIIgenetika; 1, Dorozhny Proezd., 1, 113545, Moscow, Russia, since September 23, 1980 under a Registration number of VKPM B-2175, and transferred from the original deposit to international deposit based on Budapest Treaty on September 1, 2000.

### (4) L-glutamic acid-producing bacteria

The L-glutamic acid producing bacteria belonging to the genus *Escherichia* may be exemplified by a strain which is resistant to L-valine, for example, *Escherichia coli* strains B11, K-12 (ATCC10798), B (ATCC11303) and W (ATCC9637).

### (5) L-isoleucine-producing bacteria

The L-isoleucine-producing bacteria belonging to the genus *Escherichia* may be exemplified by a strain TDH6/pVIC40, pMWD5 (Hashiguchi, K. et al., Biosci. Biotechnol. Biochem., 63(4), 672-679 (1999)) or AJ12919 described in EPO 685 555 A1.

### <2> Method for producing an L-amino acid

An L-amino acid can be efficiently produced by cultivating a bacterium belonging to the genus *Escherichia* which is transformed with *pycA* gene as described above and has an L-amino acid productivity in an appropriate medium, producing and accumulating the L-amino acid in the medium, and collecting the L-amino acid from the medium.

The L-amino acid is exemplified preferably by L-threonine, L-lysine, L-glutamic acid, L-homoserine, L-methionine, L-arginine, L-proline and L-isoleucine. The L-amino acid may be produced by the method of the present invention either alone or as a mixture of more than two kinds of L-amino acids.

In the method of present invention, the cultivation of the bacterium belonging to the genus *Escherichia*, the collection and purification of amino acid from the liquid medium may be performed in a manner similar to those of the conventional method for producing an amino acid by fermentation using a bacterium. A medium used in cultivation may be either a synthetic medium or a natural medium, so long as the medium includes a carbon and a nitrogen source and minerals and, if necessary, nutrients which the bacterium used requires for growth in appropriate amounts. The carbon source may include various carbohydrates such as glucose and sucrose, and various organic acids. Depending on assimilatory ability of the used bacterium, alcohol including ethanol and glycerol may be used. As the nitrogen source, ammonia, various ammonium salts such as ammonium sulfate, other nitrogen compounds such as amines, a natural nitrogen source such as peptone, soybean hydrolyzate and digested fermentative microbe are used. As minerals, monopotassium phosphate, magnesium sulfate, sodium chloride, ferrous sulfate, manganese sulfate, calcium carbonate are used.

The cultivation is preferably performed under an aerobic condition such as a shaking culture, and an aeration and stirring culture. The temperature of culture is usually 20 to 40°C, preferably 30 to 38°C. The pH of the culture is usually between 5 and 9, preferably between 6.5 and 7.2. The pH of the culture can be adjusted with ammonia, calcium carbonate, various acids, various bases, and buffers. Usually, 1 to 3-day cultivation leads to the accumulation of the target L-amino acid in the medium.

Collecting and purifiying the L-amino acid can be performed by removing solids such as cells from the medium by centrifugation or membrane filtration after cultivation, followed by ion exchange, concentration and crystalline fraction methods and the like.

### Best Mode for Carrying Out the Invention

The present invention will be more concretely explained below with reference to Examples.

### Example 1: Cloning of the pycA gene from Bacillus subtilis 168 strain

### <1> Construction of B. subtilis pycA::KmR recipient strain for cloning

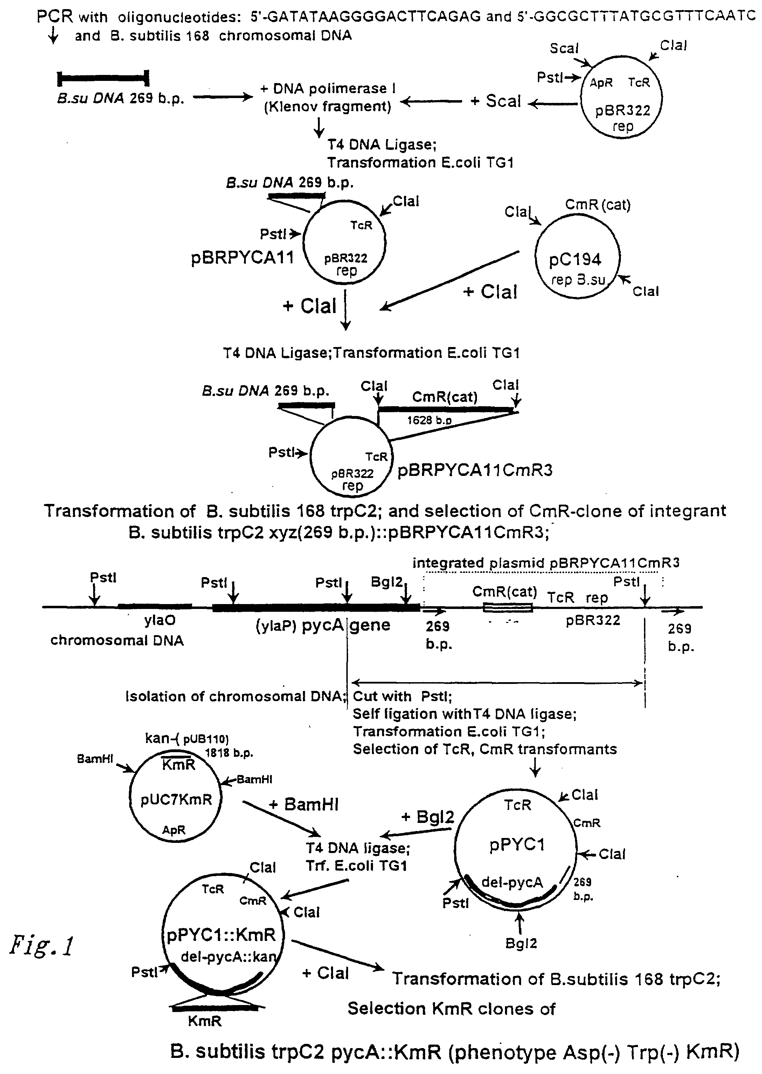

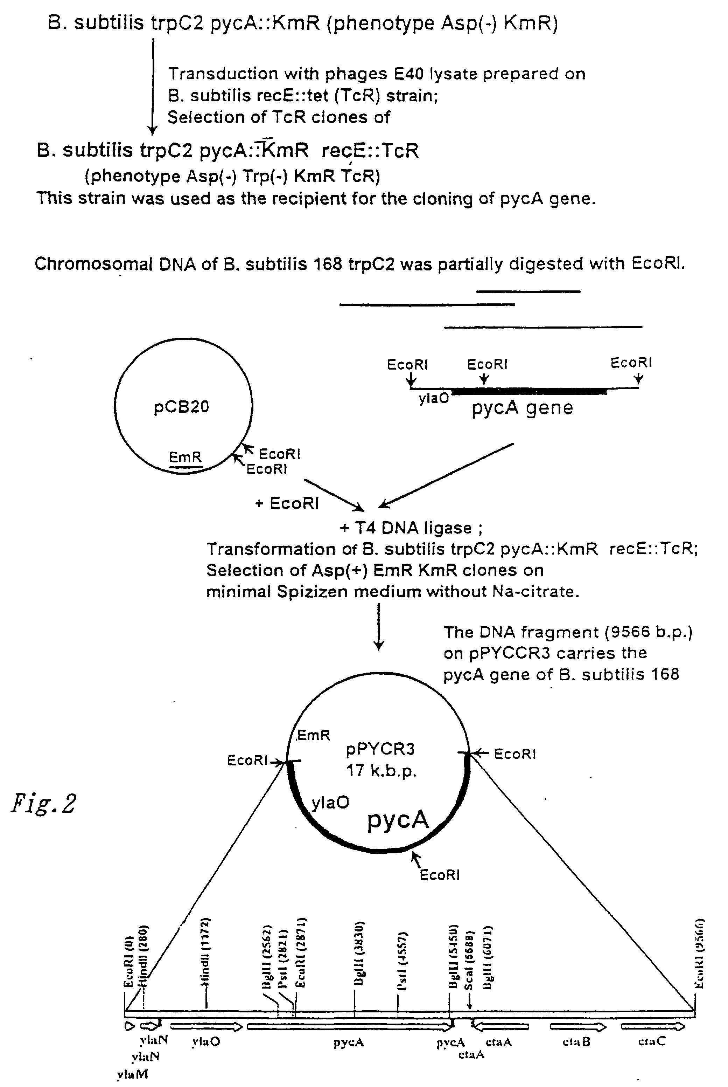

The recipient strain of *B. subtilis* was constructed by insertional mutagenesis of pycA gene. The fragment of pycA gene was cloned in two steps (refer to Figs. 1 and 2).

In a data bank one of reading frame (ylaP) from *B*. *subtilis* genome sequence project is identified as *pycA* gene, encoding pyruvate carboxylase. The region located immediately after the *pycA* gene terminator was cloned by PCR amplification, using the following pair of oligonucleotides: and

The 269bp DNA fragment was blunt-ended with Klenow fragment of DNA polymerase I and cloned into *Sca*I site of pBR322 plasmid in *E*. *coli* strain. The resulting plasmid pBRPYCA11 was digested by *Cla*I and ligated to 1628bp DNA fragment carrying cat-gene, which was obtained by digestion of pC194 with *Cla*I. Then, using the obtained plasmid pBRPYCA11CmR3, *Bacillus subtilis* 168 trpC2 strain was transformed and Cm^{R}-clones were selected. The plasmid pBRPYCA11CmR3 cannot autonomously replicate in baterium belonging to the genus *Bacillus*, but can integrate into chromosome near the *pycA* gene by homology with the 269bp DNA fragment. The strain was named as *B*. *subtilis* trpC2 xyz::pBRPYCACmR3. The integrant carried pBR322-replicon, *tet* gene encoding resistance to tetracycline derived from pBR322 and Cm^{R} marker.

Chromosomal DNA of *B*. *subtilis* trpC2 xyz::pBRPYCACmR3 was isolated and used to clone a distal part of *pycA* gene. The chromosomal DNA which is digested with *Pst*I was self-ligated and transformed into *E. coli* TG1 strain. Recombinants resitant to tretracycline and chloramphenicol were selected. The plasmid was recovered from one of the recombinant and named as pPYC1. The plasmid pPYC1 contained a single *Bgl*II restriction site, which located in *pycA* gene fragment. This site was used to insert 1818bp DNA fragment, containing a kanamycine resistance marker for *B. subtilis.* The plasmid pPYC1 was digested with *Bgl*II and ligated to pUC7KmR which was digested with *Bam*HI to obtain a plasmid pPYC1::KmR.

The inactivation of *pycA* gene in a wild type *B*. *subtilis* 168 trpC2 was achieved by transformation of the same strain with linearized plasmid pPYC1::Km^{R} by one step procedure, followed by selection on Luria broth supplemented with kanamycin. The Km^{R} marker was inserted into pycA gene on chromosomal DNA by homologous double-crossover recombination.

The constructed strain of *B*. *subtilis* pycA::Km^{R} trpC2 did not grow in Spizizen minimal medium (Anagnostopoulos, C and Spizizen, J., J. Bacteriol., 81, 741-746 (1961)) with tryptophan unless citrate or aspartate were added to the media.

| (Composition of Spizizen minimal medium) | |
|---|---|
| K₂HPO₄·3H₂O | 18.3 g/L |
| KH₂PO₄ | 6.0 g/L |
| (NH4)₂SO₄ | 2.0 g/L |
| sodium citrate·5H₂O | 1.2 g/L |
| MgSO₄·7H₂O | 0.2 g/L |
| Glucose | 10.0 g/L |
| pH7.0 | |

The *B*. *subtilis* trpC2 pycA::Km^{R} recE::Tc^{R} strain was constructed by transducing the inactivated allele of *recE* gene from *B. subtilis* recE::Tc^{R} strain with phage E40 (Fig. 2). It was necessary to inactivate recombination in recipient strain for cloning of *pycA* gene from *B. subtilis*. The constructed strain *B*. *subtilis* trpC2 pycA::Km^{R} recE::Tc^{R} strain was used as recipient to clone pycA gene.

### <2> Cloning of B. subtilis native pycA gene by complementation.

The *pycA* gene was cloned from *B. subtilis* 168 trpC2 strain. Chromosomal DNA prepared from the strain 168 trpC2 was partially digested with *Eco*RI and ligated to pCB20 (EmR) (gifted by Dr. Yu Jomantas, refer to "Genetic transformation and expression " L.O. Butler, et al. eds., Intercept Ltd., PO Box 402, Wimborne, Dorset, BH229T2, U.K., 1989, p269-281) which was digested with *Eco*RI. Then the trpC2 pycA::Km^{R} recE::Tc^{R} recipient strain was transformed with the ligation mixture (Fig. 2). The Asp⁺,Em^{R},Km^{R} clones were selected on Spizizen minimal medium without citrate or aspartate supplementation but suplemented with tryptophan, erythromycin and kanamycin. The plasmid pPYCR3 harbored by one of the selected clones complemented *pycA* mutation, had a predicted structure and conferred the same Asp⁺, Em^{R} phenotype after second transformation.

### <3> Cloning of DNA fragment carrying pycA gene from B. subtilis 168 trpC2 strain into high copy number plasmid pUC18 and pUC19

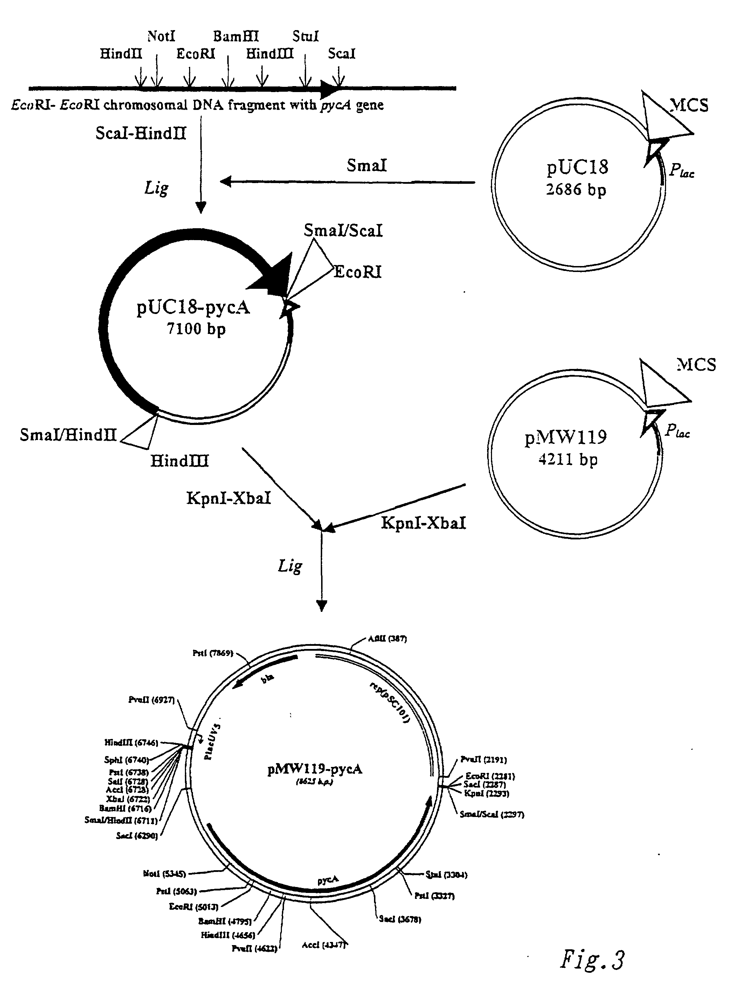

Plasmid pPYCR3 was digested with *Hin*dII and *Sca*I and *Hin*dII-*Sca*I fragment (4414 bp), carrying *pycA* gene with 5'-end upstream sequence (including promoter and ribosome binding site), was cloned in pUC18 digested with *Sma*I. Thus the plasmid pUC18-pycA was obtained (Fig. 3, upside). The ligation reaction was performed concretely as follows. Ninety ng of *Sma*I-digested pUC18 and 300 ng of *Hind*II-*Sca*I fragment (4414 bp) carrying pycA gene which was purified by agarose gel electrophresis were ligated with 2 units of T4 DNA Ligase (Pharmacia, Sweden) in 45 µl of reaction mixture containing 1x One-Phor-All buffer (Pharmacia, Sweden) and 1 mM ATP.

*E. coli* TG1 was transformed with pUC18-pycA. Obtained transformants were analyzed by using *Kpn*I, *Xba*I, *Bam*HI, *Hin*dIII and *Eco*RI endonucleases. All clones carried the *pycA* gene in opposite direction to *lac* promoter of pUC18 plasmid. However, the *pycA* genes cloned in pUC18 may be expressed under the control of the inherent promoter of the gene.

Then in order to rearrange *pycA* gene under control of *lac* promoter, the *Kpn*I-*Xba*I fragment of pUC18-pycA was cloned into pUC19 digested with *Kpn*I-*Xba*I. However, the obtained clones were unstable and the recombinant plasmid was eliminated during short time (6-8 h) of cultivation.

### <4> Cloning of DNA fragment carrying pycA gene into a low copy number plasmid pMW119

The *Kpn*I-*Xba*I fragment of pUC18-pycA was ligated to a low copy number plasmid pMW119 digested with *Kpn*I and XbaI (Fig. 3, downside). The ligation reaction was performed in 50 µl of reaction mixture containing 60 ng of pMW119 digested with *Kpn*I-*Xba*I, 120 ng of *Kpn*I-*Xba*I fragment of pUC18-pycA which was purified by agarose gel electrophresis, 1x One-Phor-All buffer (Pharmacia, Sweden), 1 mM ATP and 1 unit of T4 DNA Ligase (Pharmacia, Sweden).

Using the ligation mixture, *E. coli* TG1 was transformed. Resulting transformants were analyzed using *Sac*I, *Kpn*I, *Xba*I, *Bam*HI, *Hind*III and *Eco*RI. All clones carried *pycA* gene under control of *lac* promoter of pMW119 and its own promoter. The thus obtained clone was named TG1(pMW119-pycA), from which the plasmid pMW119-pycA was obtained.

### Example 2: Production of L-threonine and L-glutamic acid

E. coli strain MG442 was transformed with the plasmid pMW119-pycA and 10 Amp^{r} colonies were sellected. Productivities of L-threonine and L-glutamic acid of the colonies were examined using the following fermentation midium.

| (Composition of fermentation medium) | |
|---|---|
| glucose (separately sterilized) | 60 g/L |
| (NH4)₂SO₄ | 15.0 g/L |
| KH₂PO₄ | 1.5 g/L |
| MgSO₄·7H₂O | 1.0 g/L |
| L-isoleucine | 100 mg/L |
| thiamine | 0.1 mg/L |
| CaCO₃ (separately sterilized) | 20 g/L |

Fermentation was performed in test tubes (20x300mm) at 32 °C for 72 hours using a rotary shaker. One loop of each of 10 clones was inoculated into the test tube containing 2.0 ml of the fermentation medium. As controls, 10 single colonies of a plasmid-less strain MG442 were used. After the fermention, concentrations of L-threonine and L-glutamic acid accumulated in the media were mesured by thin-layer chromatography. The results are shown in Table 1.

**Table 1**

| Clone Nos. | MG442(control) | | MG442(pMW119-pycA) | |
|---|---|---|---|---|
| | L-thr G/L | L-glu G/L | L-thr g/L | L-glu g/L |
| 1 | 5.5 | 9.5 | 7.8 | 12.3 |
| 2 | 3.2 | 7.1 | 9.8 | 12.3 |
| 3 | 3.6 | 7.1 | 9.4 | 13.3 |
| 4 | 3.4 | 8.9 | 9.8 | 11.8 |
| 5 | 3.3 | 8.9 | 9.8 | 11.8 |
| 6 | 4.8 | 7.3 | 9.9 | 11.8 |
| 7 | 3.3 | 7.0 | 10.0 | 10.7 |
| 8 | 4.7 | 8.3 | 10.0 | 10.4 |
| 9 | 3.2 | 7.3 | 9.7 | 11.2 |
| 10 | 2.6 | 4.1 | 10.0 | 10.7 |
| Average | 3.8 | 7.4 | 9.6 | 11.6 |

### Example 3: Production of L-homoserine

L-homoserine-producing E. coli strain 44 was transformed with the plasmid pMW119-pycA and five ampiciline resistant clones were selected. Production of L-homoserine was examined as it is indicated in the Example 1, but the fermentation medium was supplemented with 0.5 g/l of L-threonine. As a control five colonies of plasmidless strain 44 were used. After the fermentation average L-homoserine concentrations were 4.5 g/l (the strain 44) and 5.7 g/l (the strain 44 with the plasmid pMW119-pycA).

Also the effect of pycA gene on the productivity of L-lysine is reasonably expected, because pycA gene is confirmed to have the positive effect on the productivity of L-threonine and L-homoserine which share the same biosynthetic pathway in part as L-lysine.

## Claims

1. A bacterium belonging to the genus Escherichia which is transformed with a gene coding for pyruvate carboxylase and has L-amino acid productivity,
wherein the pyruvate carboxylase comprises the amino acid sequence of SEQ ID NO: 4.

2. A bacterium belonging to the genus *Escherichia* which is transformed with a gene coding for pyruvate carboxylase and has L-amino acid productivity, wherein the gene comprises the nucleotide sequence of SEQ ID NO: 3, or the gene is obtainable from the chromosomal DNA of bacterium belonging the genus *Bacillus* by PCR with oligonucleotide primers based on the nucleotide sequence of SEQ ID NO:3.

3. The bacterium according to claim 1 or 2, wherein the gene coding for pyruvate carboxylase is introduced into said bacterium in a low copy number.

4. The bacterium according to any of the preceding claims, which produces a higher amount of the L-amino acid than the bacterium not containing said gene.

5. The bacterium according to any of the preceding claims, which produces L-threonine in a concentration which is at least 100 % higher than the concentration of L-threonine produced by a bacterium not containing said gene.

6. The bacterium according to any of the preceding claims, which produces L-glutamic acid in a concentration which is at least 50 % higher than the concentration of L-glutamic acid produced by a bacterium not containing said gene.

7. The bacterium according to any of the preceding claims, which produces L-homoserine in a concentration which is at least 25 % higher than the concentration of L-homoserine produced by a bacterium not containing said gene.

8. The bacterium having the accession number of VKPM B-7821.

9. The bacterium having the accession number of VKPM B-7822.

10. A method for producing L-amino acid comprising cultivating the bacterium according to any of the claims 1 to 9 in a medium, producing and accumulating L-amino acid in the medium, and collecting the L-amino acid from the medium.

## Patentansprüche

1. Bakterium der Gattung Escherichia, welches mit einem Gen transformiert ist, das für eine Pyruvatcarboxylase kodiert, und L-Aminosäureproduktivität hat,
wobei die Pyruvatcarboxylase die Aminosäuresequenz der SEQ ID NO:4 enthält.

2. Bakterium der Gattung Escherichia, welches mit einem Gen transformiert ist, das für eine Pyruvatcarboxylase kodiert, und L-Aminosäureproduktivität hat,
wobei das Gen die Nucleotidsequenz der SEQ ID NO:3 enthält oder das Gen aus der chromosomalen DNA eines Bakteriums der Gattung Bacillus durch PCR mit Oligonucleotidprimern, die auf der Nucleotidsequenz der SEQ ID NO:3 basieren, erhältlich ist.

3. Bakterium nach Anspruch 1 oder 2, wobei das Gen, welches für die Pyruvatcarboxylase kodiert, in einer geringen Kopienzahl in das Bakterium eingebracht ist.

4. Bakterium nach einem der vorhergehenden Ansprüche, welches eine größere Menge an L-Aminosäure produziert als das Bakterium, welches das Gen nicht enthält.

5. Bakterium nach einem der vorhergehenden Ansprüche, welches L-Threonin in einer Konzentration produziert, die mindestens 100 % höher als die Konzentration an L-Threonin ist, die durch ein Bakterium produziert wird, welches das Gen nicht enthält.

6. Bakterium nach einem der vorhergehenden Ansprüche, welches L-Glutaminsäure in einer Konzentration produziert, die mindestens 50 % höher als die Konzentration an L-Glutaminsäure ist, die durch ein Bakterium produziert wird, welches das Gen nicht enthält.

7. Bakterium nach einem der vorhergehenden Ansprüche, welches L-Homoserin in einer Konzentration produziert, die mindestens 25 % höher als die Konzentration von L-Homoserin ist, die durch ein Bakterium produziert wird, welches das Gen nicht enthält.

8. Bakterium mit der Hinterlegungsnummer VKPM B-7821.

9. Bakterium mit der Hinterlegungsnummer VKPM B-7822.

10. Verfahren zur Herstellung einer L-Aminosäure, welches das Kultivieren des Bakteriums nach einem der Ansprüche 1 bis 9 in einem Medium, das Herstellen und Akkumulieren der L-Aminosäure in dem Medium und das Gewinnen der L-Aminosäure aus dem Medium umfasst.

## Revendications

1. Bactérie appartenant au genre Escherichia qui est transformée avec un gène codant pour une pyruvate carboxylase et a une productivité en acide aminé de forme L,
dans laquelle la pyruvate carboxylase comprend la séquence d'acides aminés de SEQ ID N°: 4.

2. Bactérie appartenant au genre Escherichia qui est transformée avec un gène codant pour une pyruvate carboxylase et a une productivité en acide aminé de forme L, dans laquelle le gène comprend la séquence de nucléotides de SEQ ID N°: 3, ou le gène peut être obtenu à partir de l'ADN chromosomique d'une bactérie appartenant au genre Bacillus par PCR avec des amorces d'oligonucléotides basées sur la séquence de nucléotides de SEQ ID N°: 3.

3. Bactérie selon la revendication 1 ou 2, dans laquelle le gène codant pour la pyruvate carboxylase est introduit dans ladite bactérie à un nombre de copies bas.

4. Bactérie selon l'une quelconque des revendications précédentes, qui produit une quantité plus élevée de l'acide aminé sous forme L que la bactérie ne contenant pas ledit gène.

5. Bactérie selon l'une quelconque des revendications précédentes, qui produit de la L-thréonine à une concentration qui est au moins 100 % supérieure à la concentration de L-thréonine produite par une bactérie ne contenant pas ledit gène.

6. Bactérie selon l'une quelconque des revendications précédentes, qui produit de l'acide L-glutamique à une concentration qui est au moins 50 % supérieure à la concentration d'acide L-glutamique produite par une bactérie ne contenant pas ledit gène.

7. Bactérie selon l'une quelconque des revendications précédentes, qui produit de la L-homosérine à une concentration qui est au moins 25 % supérieure à la concentration de L-homosérine produite par une bactérie ne contenant pas ledit gène.

8. Bactérie ayant le numéro d'accès de VKPM B-7821.

9. Bactérie ayant le numéro d'accès de VKPM B-7822.

10. Méthode de production d'un acide aminé sous forme L comprenant les étapes consistant à cultiver la bactérie selon l'une quelconque des revendications 1 à 9 dans un milieu, à produire et laisser s'accumuler l'acide aminé sous forme L dans le milieu, et à récupérer l'acide aminé sous forme L à partir du milieu.
